# EUROPEAN PATENT APPLICATION

(11) **EP 4 702 919 A1**
(43) Date of publication of application: **04.03.2026**
(21) Application number: 24197928.5
(22) Date of filing: 02.09.2024
(51) Int. Cl.: A61B 5/087, A61B 5/1455, A61B 5/00

(54) **NASAL INTERFACE DEVICE**

(71) Applicant: Koninklijke Philips N.V., 5656 AG Eindhoven (NL)
(72) Inventor: GREENBAUM, Celeste, Eindhoven (NL); VAN LIESHOUT, Ron Martinus Laurentius, Eindhoven (NL); MUEHLSTEFF, Jens, 5656AG Eindhoven (NL); WIJSHOFF, Ralph Wilhelm Christianus Gemma Rosa, Eindhoven (NL); JANSSEN, Anton, Eindhoven (NL); GROEN, Steven Peter, Eindhoven (NL); CANNON, Ryan, Eindhoven (NL)
(74) Representative: Philips Intellectual Property & Standards

(57) **Abstract**

A nasal interface device (100) is provided. The nasal interface device includes a clamp (110) for attachment to a nasal alar, or a nasal septum, of a subject. The nasal interface device also includes a first measurement unit (120) and a second measurement unit (130). The first measurement unit (120) is mechanically coupled to the clamp (110) such that when the clamp is attached to the nasal alar, or the nasal septum, respectively, the first measurement unit (120) interfaces with the nasal alar, or the nasal septum, respectively, for measuring one or more photoplethysmography, PPG, signals for the subject. The second measurement unit (130) is configured to measure one or more physiological signals for the subject, the one or more physiological signals being signals other than a PPG signal.

## Description

### FIELD OF THE INVENTION

The present disclosure relates to the measurement of photoplethysmography, PPG, signals from a nasal alar, or a nasal septum, of a subject. A nasal interface device, and a patient monitoring system that includes the nasal interface device, are disclosed.

### BACKGROUND OF THE INVENTION

Photoplethysmography is an optical technique that measures blood volume changes in tissue. Photoplethysmography is frequently used to provide physiological information relating to the health of a subject. For instance, a PPG signal, i.e. a signal obtained by photoplethysmography, includes a pulsatile waveform arising from cardiac-induced changes in the blood volume with each heart beat. A PPG signal may therefore be used to determine the heart rate of a subject. A PPG signal may also include waveforms that represent other types of physiological information. For instance, waveforms within PPG signals have been attributed to a subject's respiration, sympathetic nervous system activity and thermoregulation. Other types of physiological information may also be derived from PPG signals. For instance blood oxygen saturation is typically derived from PPG signals that are measured at different optical wavelength intervals. Blood oxygen saturation is typically derived from the ratio of pulse amplitudes of red and infrared PPG signals (e.g. approximately 660 nm and approximately 880 nm or 940 nm, respectively) using the "ratio of ratios" technique. This metric quantifies the difference in optical absorption in tissue between oxygen-bound and oxygen-unbound haemoglobin at red versus infrared wavelengths. PPG signals may be obtained from various locations in the anatomy, including in the nasal alar, or a nasal septum, of a subject. A measurement of blood oxygen saturation obtained from the nasal alar, or a nasal septum may be referred-to as an "SpO₂ level".

However, there is an interest in obtaining further information relating to the health of a subject.

### SUMMARY OF THE INVENTION

According to one aspect of the present disclosure, a nasal interface device is provided. The nasal interface device comprises:
a clamp for attachment to a nasal alar, or a nasal septum, of a subject;
a first measurement unit; and
a second measurement unit;

The first measurement unit is mechanically coupled to the clamp such that when the clamp is attached to the nasal alar, or the nasal septum, respectively, the first measurement unit interfaces with the nasal alar, or the nasal septum, respectively, for measuring one or more photoplethysmography, PPG, signals for the subject. The second measurement unit is configured to measure one or more physiological signals for the subject, the one or more physiological signals being signals other than a PPG signal.

Since the second measurement unit measures a physiological signal/ signals other than a PPG signal, the nasal interface device provides additional information relating to the health of the subject.

According to another aspect of the present disclosure, a patient monitoring system is provided. The patient monitoring system includes a nasal interface device and a controller. The controller comprises one or more processors configured to receive the one or more PPG signals and/or the one or more physiological signals. The one or more processors may be further configured to:
output the one or more PPG signals; and/or;
calculate, and output, a blood oxygen saturation signal, or a heart rate signal, or a respiration rate signal, based on the one or more PPG signals and/or based on the one or more physiological signals; and/or
calculate, and output, a warning signal based on the one or more PPG signals and/or based on the one or more physiological signals.

The signals that are outputted in accordance with this aspect facilitate an assessment of the health of the subject. By providing the warning signal based on the PPG signal(s) and/or based on the physiological signal(s), a sensitivity, or a specificity of the warning signal may be improved.

According to another aspect of the present disclosure, a patient monitoring system is provided. The patient monitoring system includes a nasal interface device, and a controller. The nasal interface device comprises: a clamp for attachment to a nasal alar, or a nasal septum, of a subject; and a measurement unit. The measurement unit is mechanically coupled to the clamp such that when the clamp is attached to the nasal alar, or the nasal septum, of the subject, the measurement unit interfaces with the nasal alar, or the nasal septum, respectively for measuring one or more photoplethysmography, PPG, signals for the subject. The controller comprises one or more processors configured to:
receive the one or more PPG signals; and
extract, from the one or more PPG signals, a heart rate signal and/or a respiration rate signal;
calculate, and output, a warning signal based on the heart rate signal and/or the respiration rate signal.

By providing the warning signal based on the heart rate signal and/or the respiration rate signal, and which are derived from the PPG signals, the warning signal may be provided in a more straightforward manner.

According to another aspect of the present disclosure, a nasal interface device is provided. The nasal interface device comprises: a clamp for attachment to a nasal alar, or a nasal septum, of a subject; and a measurement unit. The clamp comprises: a hinge; and two prongs, The prongs are mechanically coupled to one another via the hinge. The prongs are configured to attach the clamp to the nasal alar, or the nasal septum, of the subject, respectively. The measurement unit is mechanically coupled to one or more of the prongs such that when the prongs are attached to the nasal alar, or the nasal septum, respectively, the measurement unit interfaces with the nasal alar, or the nasal septum, respectively, for measuring one or more photoplethysmography, PPG, signals for the subject. Each prong comprises a curved edge, or a bevelled edge, for sliding the prong over the nasal alar, or a nasal septum, respectively.

The curved, or bevelled, edges of the prongs of the nasal interface device may facilitate a more straightforward application of the nasal interface device to the nasal alar, or the nasal septum.

According to another aspect of the present disclosure, a nasal interface device is provided. The nasal interface device comprises: a clamp for attachment to a nasal alar, or a nasal septum, of a subject; and a measurement unit. The clamp comprises a hinge and two prongs. The prongs are mechanically coupled to one another via the hinge. The prongs are configured to attach the clamp to the nasal alar, or the nasal septum, of the subject, respectively. The measurement unit is mechanically coupled to one or more of the prongs such that when the prongs are attached to the nasal alar, or the nasal septum, respectively, the measurement unit interfaces with the nasal alar, or the nasal septum, respectively, for measuring one or more photoplethysmography, PPG, signals for the subject. Each prong further comprises a wing, the wing being mechanically coupled to the corresponding prong, and extending in an opposing direction to the prong. The wings extend to a position beyond the hinge such that when the prongs are pinched together, the wings pivot about the hinge, opening the hinge for sliding the nasal clamp over the nasal alar, or the nasal septum, respectively.

The wings of the nasal interface device may therefore facilitate a more straightforward application of the nasal interface device to the nasal alar, or the nasal septum.

Further aspects, features, and advantages of the present disclosure will become apparent from the following description of examples, which is made with reference to the accompanying drawings.

### BRIEF DESCRIPTION OF THE DRAWINGS

Fig. 1 is a schematic diagram illustrating an example of a patient monitoring system 1000 including a first example of a nasal interface device 100 with a first measurement unit 120 and a second measurement unit 130, in accordance with some aspects of the present disclosure.
Fig. 2 is a schematic diagram illustrating a second example of a nasal interface device 200 with a first measurement unit 120 and a second measurement unit 130, in accordance with some aspects of the present disclosure.
Fig. 3 is a schematic diagram illustrating a third example of a nasal interface device 300 with a first measurement unit 120, a second measurement unit 130, and a third measurement unit 150, in accordance with some aspects of the present disclosure.
Fig. 4 is a schematic diagram illustrating an example of a patient monitoring system 1000 including a fourth example of a nasal interface device 400 with a first measurement unit 120 and a second measurement unit 130, in accordance with some aspects of the present disclosure.
Fig. 5 is a schematic diagram illustrating an example of a patient monitoring system 1000 including a fifth example of a nasal interface device 500 with a first measurement unit 120 and a second measurement unit 130, in accordance with some aspects of the present disclosure.
Fig. 6 is a schematic diagram illustrating an example of a patient monitoring system 1000 including a sixth example of a nasal interface device 600 with a first measurement unit 120 and a second measurement unit 130, in accordance with some aspects of the present disclosure.
Fig. 7 is a schematic diagram illustrating an example of a nasal interface device 700 with a measurement unit 120 and two prongs 110b, 110c, that include curved edges for sliding over a nasal alar, or a nasal septum, in accordance with some aspects of the present disclosure.
Fig. 8 is a schematic diagram illustrating an example of a nasal interface device 800 with a measurement unit 120 and two prongs 110b, 110c, that include bevelled edges for sliding over a nasal alar, or a nasal septum, in accordance with some aspects of the present disclosure.
Fig. 9 is a schematic diagram illustrating an example of a nasal interface device 900 with a measurement unit 120 and wings 110"b, 110"c, for use in sliding over a nasal alar, or a nasal septum, in accordance with some aspects of the present disclosure.

### DETAILED DESCRIPTION OF EMBODIMENTS

Examples of the present disclosure are provided with reference to the following description and figures. In this description, for the purposes of explanation, numerous specific details of certain examples are set forth. Reference in the specification to "an example", "an implementation" or similar language means that a feature, structure, or characteristic described in connection with the example is included in at least that one example. It is also to be appreciated that features described in relation to one example may also be used in another example, and that all features are not necessarily duplicated in each example for the sake of brevity. For instance, features described in relation to one example of a nasal interface device may be implemented in another example of a nasal interface device, in a corresponding manner. Similarly, features described in relation to an example of a nasal interface device may be included in examples of patient monitoring systems.

In the following description, reference is made to the measurement of various physiological signals by various measurement units. In some examples, reference is made to the measurement, by the measurement units, of physiological signals such as a PPG signal, a (core body) temperature signal representing a temperature of the subject, a temperature signal representing a temperature of exhaled breath of the subject, an air flow signal representing a respiration rate of the subject, and a (end-tidal, "etCO₂") carbon dioxide signal representing a carbon dioxide content of exhaled breath of the subject. It is, however to be noted that unless explicitly stated, the measurement units described herein may alternatively be used to measure other types of physiological signals.

The explicit use of the terms "processor" or "controller" should not be interpreted as exclusively referring to hardware capable of running software, and can implicitly include, but is not limited to, digital signal processor "DSP" hardware, read only memory "ROM" for storing software, random access memory "RAM", a non-volatile storage device, and the like. Where reference is made to a processor, it is noted that the processor may be provided a single dedicated processor, or by a single shared processor, or by a plurality of individual processors, some of which can be shared. The function(s) of the processor may for instance be provided by processors that are shared within a networked processing architecture such as a client/server architecture, a peer-to-peer architecture, the Internet, or the Cloud.

Furthermore, examples of the present disclosure can take the form of a computer program product accessible from a computer-usable storage medium, or a computer-readable storage medium, the computer program product providing program code for use by or in connection with a computer or any instruction execution system. For the purposes of this description, a computer-usable storage medium or a computer readable storage medium can be any apparatus that can comprise, store, communicate, propagate, or transport a program for use by or in connection with an instruction execution system, apparatus, or device. The medium can be an electronic, magnetic, optical, electromagnetic, infrared, or a semiconductor system or device or propagation medium. Examples of computer-readable media include semiconductor or solid-state memories, magnetic tape, removable computer disks, random access memory "RAM", read-only memory "ROM", rigid magnetic disks and optical disks. Current examples of optical disks include compact disk-read only memory "CD-ROM", compact disk-read/write "CD-R/W", Blu-Ray^{™} and DVD.

It is also noted that some operations that are described as being performed by the one or more processors of the system disclosed herein may be implemented using artificial intelligence techniques. Suitable techniques may include machine learning techniques, deep learning techniques, and neural networks. For instance, one or more neural networks, may be trained in a supervised, or in some cases unsupervised, manner, to implement the operations that are performed by the one or more processors.

As mentioned above, there is an interest in obtaining further information relating to the health of a subject.

Some examples of the present disclosure relate to a nasal interface device. Other examples of the present disclosure relate to a patient monitoring system that includes a nasal interface device. In general, the nasal interface device may be provided alone, or in combination with a patient monitoring system.

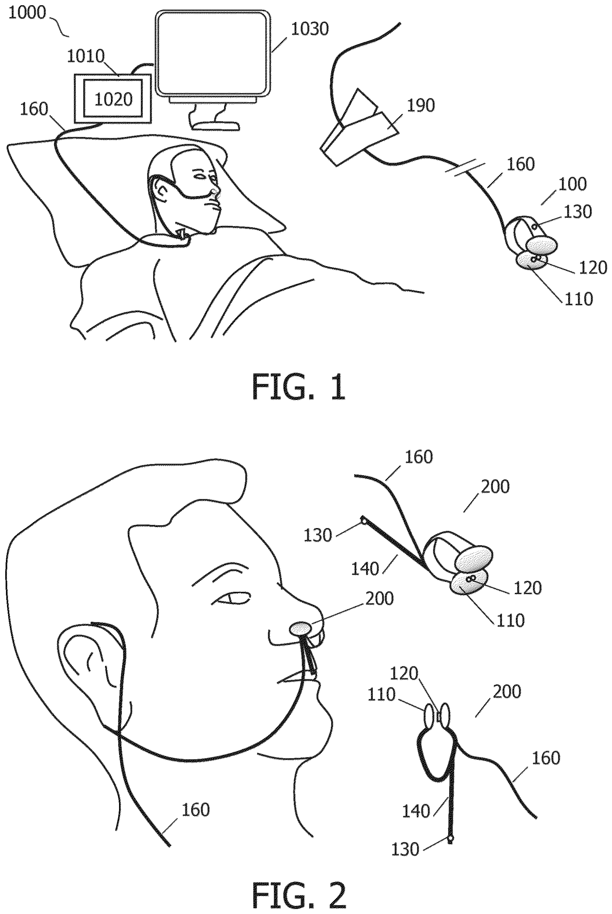

Fig. 1 is a schematic diagram illustrating an example of a patient monitoring system 1000 including a first example of a nasal interface device 100 with a first measurement unit 120 and a second measurement unit 130, in accordance with some aspects of the present disclosure. The nasal interface device 100 is illustrated in greater detail on the right-hand side of Fig. 1. The nasal interface device 100 includes a clamp 110 for attachment to a nasal alar, or a nasal septum, of a subject. As illustrated in Fig. 1, the clamp may include two prongs that are coupled to one another by a hinge. The hinge may provide a restoring force that counteracts a deformation of the hinge from a relaxed state of the hinge. The hinge may be formed from, or include, a flexible, or elastic, material such as a polymer, or a metal, for example. The nasal interface device 100 also includes a first measurement unit 120 and a second measurement unit 130. The first measurement unit 120 is mechanically coupled to the clamp 110 such that when the clamp is attached to the nasal alar, or the nasal septum, respectively, the first measurement unit 120 interfaces with the nasal alar, or the nasal septum, respectively, for measuring one or more photoplethysmography, PPG, signals for the subject. The second measurement unit 130 is configured to measure one or more physiological signals for the subject, the one or more physiological signals being signals other than a PPG signal.

In this example, since the second measurement unit measures a physiological signal/ signals other than a PPG signal, the nasal interface device provides additional information relating to the health of the subject.

Various types of measurement units are contemplated for use as the measurement units 120, 130 in Fig. 1.

The first measurement unit 120 illustrated in Fig. 1 measures one or more PPG signals for the subject. In general, the first measurement unit 120, may include at least one optical source and at least one optical detector; and wherein the at least one optical source and at least one optical detector are configured to measure one or more of the following optical parameters for a portion of the nasal alar, or a portion of the nasal septum, respectively: an optical transmission, an optical reflectance, an optical absorption, and an optical scattering.

Various arrangements of optical source(s) and optical detector(s) may be used in the first measurement unit 120. For instance, in some arrangements, an optical source and an optical detector are disposed on the clamp such that when the clamp is attached to the nasal alar, or the nasal septum, the optical source optical detector are disposed on opposite sides of the nasal alar or the nasal septum. Such arrangements may be used to measure an optical transmission or an optical absorption of the nasal alar or the nasal septum. In other arrangements, an optical source and an optical detector are disposed on the clamp such that when the clamp is attached to the nasal alar, or the nasal septum, the optical source and the optical detector are disposed on a same side of the nasal alar or the nasal septum. Such arrangements may be used to measure an optical reflectance, or an optical scattering. Combinations of these arrangements may be used to measure one or more of: the optical transmission, the optical reflectance, the optical absorption, and the optical scattering, of the nasal alar or the nasal septum. In some arrangements, one or more optical parameters are measured within wavelength intervals that correspond to different portions of the optical spectrum. For instance, in one example, a first PPG signal is measured within a wavelength interval corresponding to a red portion of the optical spectrum, and a second PPG signal is measured within a wavelength interval corresponding to an infrared portion of the optical spectrum. The first PPG signal may be measured within a wavelength interval that includes a wavelength of approximately 660 nanometres, and the second PPG signal may be measured within a wavelength interval that includes a wavelength of approximately 880 nm or 940 nanometres. Such PPG signals may be used to calculate blood oxygen saturation. For example, blood oxygen saturation is typically calculated using the "ratio of ratios" technique based on the ratio of pulse amplitudes of red and infrared signals (e.g. 660 nm and 880 nm or 940 nm, respectively). A measurement of the blood oxygen saturation obtained from the nasal alar, or the nasal septum may be referred-to as an "SpO₂ level".

As illustrated in Fig. 1, the first measurement unit 120 may also include one or more electrical conductors 160, or one or more optical waveguides. The electrical conductor(s), or optical waveguide(s) may be used to communicate signals to the first measurement unit for generating the one or more PPG signals, and to communicate the one or more PPG signals generated in response to the signals. The electrical conductor(s), or optical waveguide(s) may be used to communicate signals from a controller 1010 to the first measurement unit for generating the one or more PPG signals, and to communicate back to the controller the one or more PPG signals generated in response to the signals. As also illustrated in Fig. 1, the electrical conductor(s), or optical waveguide(s) may be provided with a clip 190 to facilitate attachment to an item of clothing.

As mentioned above, the second measurement unit 130 illustrated in Fig. 1 measures one or more physiological signals for the subject other than a PPG signal. In general, the second measurement unit 130 may include at least one source and/or at least one detector. Examples of physiological signals that may be measured by the second measurement unit 130 include a temperature signal representing a (core body) temperature of the subject, a temperature signal representing a temperature of exhaled breath of the subject, an air flow signal representing a respiration rate of the subject, and a carbon dioxide signal representing a carbon dioxide content of exhaled breath of the subject.

Various types, and also arrangements, of sources and detectors may be used in the second measurement unit 130. For instance, temperature signals may be measured using a temperature sensor. Various types of temperature sensors may be used as the temperature sensor, including for example a thermistor, a thermocouple, and an infrared temperature sensor. An air flow signal may be measured using a gas flow sensor, and carbon dioxide signals may be measured using a carbon dioxide detector. The use of other types of detectors and/or sources is also contemplated. For instance, in one example, the use of an optical source (such as a laser or a light emitting diode "LED") and an optical detector (such as a photodetector) is contemplated. In some examples, the use of an optical source that delivers optical radiation via one or more optical fibers, is contemplated. In some examples, and which may be combined with the former example, the use of an optical detector that detects collected optical radiation via one or more optical fibers, is contemplated. In some examples, the optical source(s) and/or optical detector(s) are disposed in the controller 1010 and the optical fiber(s) guide optical radiation to and from the distal ends of the optical fiber(s), the latter serving as the second measurement unit. In some examples, the source may include an electrical source, and the detector may include an electrical detector. For instance, an electrical source and an electrical detector may be arranged to provide a physiological signal in the form of a (galvanic) skin resistance measurement.

With continued reference to Fig. 1, the first measurement unit 120 is configured to interface with the nasal alar, or the nasal septum. In the example illustrated in Fig. 1, the first measurement unit 120 is coupled to a prong of the clamp 110 of the nasal interface device. Thus, when the clamp 110 is attached to the nasal alar, or the nasal septum, the first measurement unit 120 interfaces with the nasal alar or the nasal septum, respectively.

The second measurement unit 130 is configured to measure one or more physiological signals for the subject. The second measurement unit may be disposed in various locations, as illustrated in the examples of the nasal interface devices shown in Fig. 1 - Fig. 6.

In the example illustrated in Fig. 1, the second measurement unit is mechanically coupled to the clamp such that when the clamp is attached to the nasal alar, or the nasal septum, respectively, the second measurement unit is positioned in a nasal airflow of the subject for measuring the one or more physiological signals from the nasal airflow of the subject. In the example illustrated in Fig. 1, the second measurement unit 130 is mechanically coupled to a prong of the clamp. In some examples, the second measurement unit 130 is mechanically coupled to a different prong of the clamp to the one or more electrical conductors 160, or one or more optical waveguides. For instance, in situations in which the clamp is attached to the nasal alar, the one or more electrical conductors 160, or one or more optical waveguides are less cumbersome if they are attached to the prong that remains outside of the nostril. In this situation, mechanically coupling the second measurement unit 130 to the opposite prong ensures that the second measurement unit 130 will be disposed within a nostril when the clamp is attached to the nasal alar, or the nasal septum. The second measurement unit 130 may alternatively be mechanically coupled to another portion of the clamp such that when the clamp is attached to the nasal alar, or the nasal septum, respectively, the second measurement unit is positioned in a nasal airflow of the subject. For instance, the second measurement unit 130 may alternatively be mechanically coupled to a hinge of the clamp.

Fig. 2 is a schematic diagram illustrating a second example of a nasal interface device 200 with a first measurement unit 120 and a second measurement unit 130, in accordance with some aspects of the present disclosure. Items in Fig. 2 that share the same labels as items in Fig. 1 provide the same functionality and this is not duplicated for sake of brevity. The nasal interface device 200 illustrated in Fig. 2 differs from the nasal interface device 100 illustrated in Fig. 1 in that the second measurement unit of the nasal interface device 200 is mechanically coupled to the clamp such that when the clamp is attached to the nasal alar, or the nasal septum, respectively, the second measurement unit is positioned in an oral airflow of the subject for measuring the one or more physiological signals from the oral airflow of the subject.

In the nasal interface device 200 illustrated in Fig. 2, the nasal interface device 200 includes an arm 140. The arm comprises a proximal portion and a distal portion. The proximal portion of the arm is mechanically coupled to the clamp. The second measurement unit is arranged on the distal portion of the arm such that when the clamp is attached to the nasal alar, or the nasal septum, respectively, the second measurement unit is positioned in an oral airflow of the subject, for measuring the one or more physiological signals from the oral airflow of the subject.

In the nasal interface device 200 illustrated in Fig. 2, the arm extends towards the mouth of the subject for measuring the one or more physiological signals from the oral airflow of the subject. A similar arrangement that includes an arm may be used to position the second measurement unit in the nasal airflow of the subject. In this case, the nasal interface device includes an arm 140. In general, the arm may be formed from flexible material, or a rigid material, or a malleable material. The arm may have various shapes, such as for example the straight shape illustrated in Fig. 2, or a curved, or angular shape. The arm comprises a proximal portion and a distal portion. The proximal portion of the arm is mechanically coupled to the clamp. The second measurement unit is arranged on the distal portion of the arm such that when the clamp is attached to the nasal alar, or the nasal septum, respectively, the second measurement unit is positioned in a nasal airflow of the subject, for measuring the one or more physiological signals from the nasal airflow of the subject. Improved positioning, and hence physiological measurements of the nasal, or oral, airflow, may be obtained by using the arm 140.

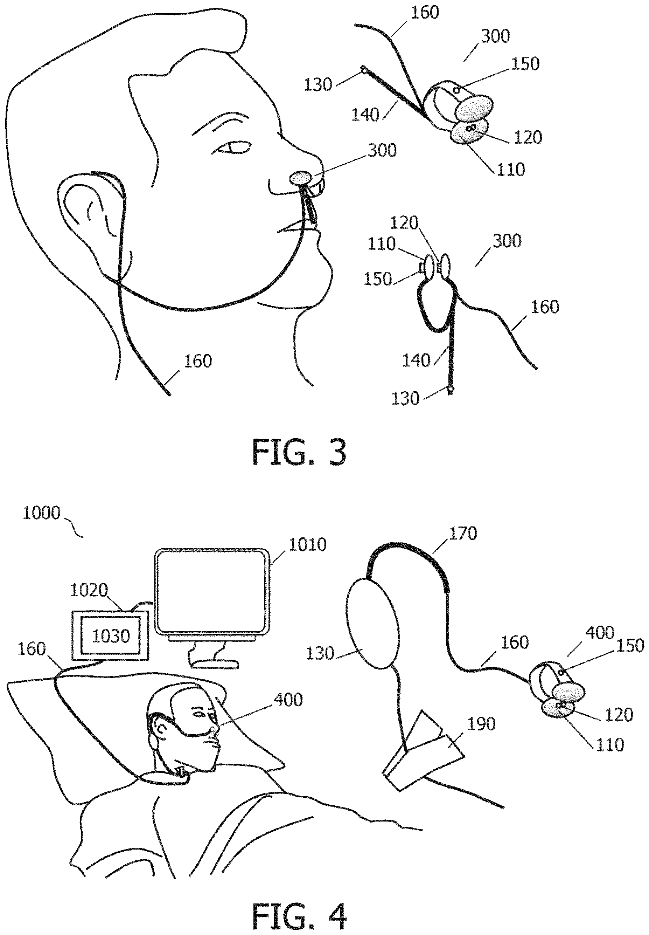

Fig. 3 is a schematic diagram illustrating a third example of a nasal interface device 300 with a first measurement unit 120, a second measurement unit 130, and a third measurement unit 150, in accordance with some aspects of the present disclosure. Items in Fig. 3 that share the same labels as items in Fig. 2 provide the same functionality and this is not duplicated for sake of brevity. The nasal interface device 300 illustrated in Fig. 3 differs from the nasal interface device 100 illustrated in Fig. 2 in that the nasal interface device 300 further includes a third measurement unit 150. The third measurement unit is configured to measure one or more physiological signals for the subject, the one or more physiological signals being signals other than a PPG signal. The third measurement unit is mechanically coupled to the clamp such that when the clamp is attached to the nasal alar, or the nasal septum, respectively, the third measurement unit is positioned in a nasal airflow of the subject for measuring the one or more physiological signals from the nasal airflow of the subject. The third measurement unit 150 may measure any of the physiological signals described above in relation to the second measurement unit 130. The third measurement unit 150 may be provided in a similar manner to the second measurement unit 130 described above. The use of both the second and the third measurement units provides additional information relating to the subject. Moreover, it may provide redundancy. For instance, the arrangement illustrated in Fig. 3 is capable of measuring physiological parameters in both the nasal airflow and the oral airflow of the subject. This arrangement can therefore provide physiological signals in spite of a change in a subject's breathing pattern between oral-breathing and nasal-breathing, or in spite of a reduction in the nasal or oral airflow of the subject.

Fig. 4 is a schematic diagram illustrating an example of a patient monitoring system 1000 including a fourth example of a nasal interface device 400 with a first measurement unit 120 and a second measurement unit 130, in accordance with some aspects of the present disclosure. Items in Fig. 4 that share the same labels as items in Fig. 1 provide the same functionality and this is not duplicated for sake of brevity. The nasal interface device 400 illustrated in Fig. 4 differs from the nasal interface device 100 illustrated in Fig. 1 in that the nasal interface device 400 also includes an ear attachment 170 for attachment to an ear of the subject. The ear attachment 170 illustrated in Fig. 4 is provided in the form of an ear hook. The ear attachment 170 may alternatively be provided in another form, such as a loop. The ear attachment may be provided by a material such as a polymer. The ear hook attaches around a portion of the ear of the subject. The nasal interface device 400 illustrated in Fig. 4 also differs from the nasal interface device 100 illustrated in Fig. 1 in that the second measurement unit 130 of the nasal interface device 400, is mechanically coupled to the ear attachment rather than being arranged on the clamp 110.

In the nasal interface device 400 illustrated in Fig. 4, the first measurement unit 120 comprises one or more electrical conductors 160, or one or more optical waveguides, configured to communicate signals to the first measurement unit for generating the one or more PPG signals, and to communicate the one or more PPG signals generated in response to the signals. The nasal interface device 400 further comprises an ear attachment 170 for attachment to an ear of the subject. The one or more electrical conductors 160, or the one or more optical waveguides, are mechanically coupled to the ear attachment 170 and the ear attachment is configured to guide the respective one or more electrical conductors, or the one or more optical waveguides, around a portion of the ear of the subject. The second measurement unit 130 is mechanically coupled to the ear attachment such that when the ear attachment is attached to the ear of the subject, the second measurement unit interfaces with a region of the head of the subject, for measuring the one or more physiological signals for the subject based on a temperature of the region of the head.

The second measurement unit 130 in this example may include one or more temperature sensors. Various types of temperature sensors may be used as the temperature sensor, including for example a thermistor, a thermocouple, and an infrared temperature sensor. The second measurement unit 130 may be arranged to measure a temperature of a region of the head such as a temperature of the skin covering the mastoid process. This region provides a suitable location for measuring the temperature of blood in the region of the posterial auriular artery. The second measurement unit 130 may alternatively be arranged to measure a temperature of another region of the head such as a temperature of the skin covering the temple. This region provides a suitable location for measuring the temperature of blood in the region of the temporal artery. These temperature measurements may serve as surrogates for the core body temperature of the subject. The temperature may alternatively be measured at other regions of the head. In the example illustrated in Fig. 4, a head interface portion of the second measurement unit 130 may include an adhesive material for improving the thermal coupling between the second measurement unit 130 and the region of the head at which the temperature is measured. The head interface portion of the second measurement unit 130 may also include a material such as a foam for providing patient comfort and/or improving thermal coupling.

The physiological signals that are measured in accordance with this example include the (core) body temperature. Thus, it provides additional information relating to the health of the subject.

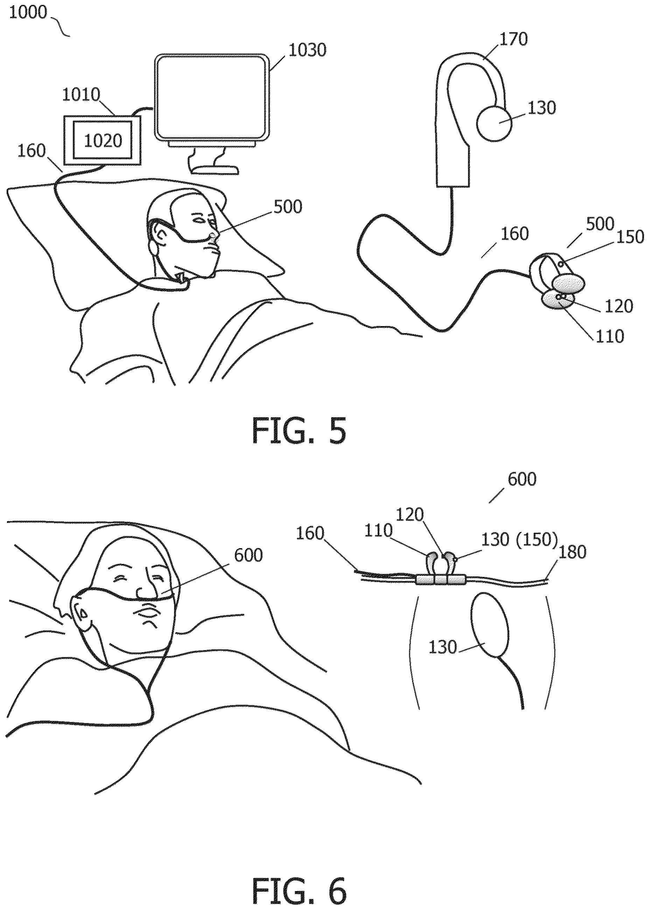

Fig. 5 is a schematic diagram illustrating an example of a patient monitoring system 1000 including a fifth example of a nasal interface device 500 with a first measurement unit 120 and a second measurement unit 130, in accordance with some aspects of the present disclosure. Items in Fig. 5 that share the same labels as items in Fig. 4 provide the same functionality and this is not duplicated for sake of brevity. The nasal interface device 500 illustrated in Fig. 5 differs from the nasal interface device 400 illustrated in Fig. 4 in that the second measurement unit 130 of the nasal interface device 500 interfaces with a region of the ear of the subject for measuring the one or more physiological signals for the subject based on a temperature of the region of the ear.

In general, the second measurement unit 130 of the nasal interface device 500 illustrated in Fig. 5 may contact the region of the ear, or it may be separated from the region of the ear such that the second measurement unit receives thermal radiation from the region of the ear. In the latter case, the second measurement unit 130 may include an infrared radiation sensor that is configured to receive the thermal radiation from the tympanic membrane of the ear of the subject. This location provides a reliable measurement of the (core body) temperature of the subject.

It is noted that the nasal interface device 400 illustrated in Fig. 4 and the nasal interface device 500 illustrated in in Fig. 5 may also include a third measurement unit 150 that is mechanically coupled to the clamp such that when the clamp is attached to the nasal alar, or the nasal septum, respectively, the third measurement 150 unit is positioned in a nasal airflow of the subject for measuring the one or more physiological signals from the nasal airflow of the subject. It is noted that whilst the third measurement unit 150 is illustrated in Fig. 4 and in Fig. 5, its use is optional. If the third measurement unit 150 is included, it may provide the functionality, and be provided in the same way as that described above in relation to the third measurement unit 150 in Fig. 3.

In some examples, the nasal interface device is provided with a cannula for delivering a gas to a nasal cavity of the subject. For instance, the examples illustrated in Fig. 1 - Fig. 5 may be provided with a cannula (not illustrated). The cannula may be mechanically coupled to the clamp 110 such that when the clamp is attached to the nasal alar, or the nasal septum, respectively, a gas delivery port of the cannula is positioned in a nasal airflow of the subject for delivering the gas to the nasal cavity of the subject.

Another example nasal interface device that includes a cannula is illustrated in Fig. 6, which is a schematic diagram illustrating an example of a patient monitoring system 1000 including a sixth example of a nasal interface device 600 with a first measurement unit 120 and a second measurement unit 130, in accordance with some aspects of the present disclosure. Items in Fig. 6 that share the same labels as items in Fig. 1 provide the same functionality and this is not duplicated for sake of brevity. The nasal interface device 600 illustrated in Fig. 6 differs from the nasal interface device 100 illustrated in Fig. 1 in that the clamp 110 of the nasal interface device 600 is integrated into the cannula 180.

In this example, the nasal interface device 600 includes a cannula 180 for delivering a gas to a nasal cavity of the subject. The first measurement unit 120 comprises one or more electrical conductors 160, or one or more optical waveguides, configured to communicate signals to the first measurement unit for generating the one or more PPG signals, and to communicate the one or more PPG signals generated in response to the signals. The cannula 180 is mechanically coupled to the clamp 110, and the cannula is configured to support the one or more electrical conductors 160, or one or more optical waveguides along a path of the one or more electrical conductors, or the one or more optical waveguides, respectively. The second measurement unit 130 is mechanically coupled to the clamp, or to the cannula, such that when the clamp is attached to the nasal alar, or the nasal septum, respectively, the second measurement unit is positioned in a nasal airflow of the subject for measuring the one or more physiological signals from the nasal airflow of the subject.

Integrating the nasal interface device 600 and the cannula 180 in this manner may simplify the construction of the nasal interface device and/or provide a more straightforward interface with the subject.

In any of the examples described above, a fourth measurement unit (not illustrated) may also be provided. The fourth measurement unit may be mechanically coupled to the clamp, or to the arm, or to the ear attachment, or to the cannula of the nasal interface device. The fourth measurement unit is configured to measure a temperature of an environment of the subject. The temperature of the environment of the subject may be used to adjust the measurement temperature(s) described above in order to provide more accurate physiological signals, such as a more accurate core body temperature or a more accurate respiration rate.

In another example, a patient monitoring system 1000 is provided. The patient monitoring system 1000 may include any of the nasal interface devices 100, 200, 300, 400, 500, 600 described above, and a controller 1010. The controller comprises one or more processors 1020 configured to:
receive the one or more PPG signals and/or the one or more physiological signals; and output the one or more PPG signals; and/or;
calculate, and output, a blood oxygen saturation signal, or a heart rate signal, or a respiration rate signal, based on the one or more PPG signals and/or based on the one or more physiological signals; and/or
calculate, and output, a warning signal based on the one or more PPG signals and/or based on the one or more physiological signals.

In this example, the signals that are outputted facilitate an assessment of the health of the subject. By providing the warning signal based on the PPG signal(s) and/or based on the physiological signal(s), a sensitivity, or a specificity of the warning signal may be improved.

Examples of a nasal interface device in combination with a controller are illustrated in Fig. 1, Fig. 4, and in Fig. 5.

In this example, known techniques may be used to calculate a blood oxygen saturation signal, or a heart rate signal, or a respiration rate signal, based on the one or more PPG signals. Similarly, known techniques may be used to calculate a blood oxygen saturation signal, or a heart rate signal, or a respiration rate signal based on the one or more physiological signals. In an example, the one or more processors may implement one or more algorithms and/or one or more filters in order to extract these signals from the PPG signals/ physiological signal(s).

Examples of warning signals that may be calculated based on the one or more PPG signals and/or based on the one or more physiological signals include the so-called Paediatric Early Warning Score "PEWS", the Modified Early Obstetric Warning Score "MEOWS", the Modified Early Warning Score "MEWS", and the National Early Warning Score "NEWS/ NEWS2". Another example of the evaluation of a warning signal based on the one or more PPG signals and/or based on the one or more physiological signals is described in the document "Capnography for Monitoring End-Tidal CO2 in Hospital and Pre-hospital Settings: A Health Technology Assessment" CADTH Health Technology Assessment, No. 142. Richardson M, Moulton K, Rabb D, et al., Ottawa (ON): Canadian Agency for Drugs and Technologies in Health; 2016 Mar.

In this example, the PPG signal(s) and the physiological signal(s) may be received via any form of data communication, including via wired, or wireless, or optical fiber communication. By way of some examples, when wired data communication is used, the communication may take place via electrical signals that are transmitted on an electrical cable. When wireless data communication is used, the communication may take place via RF or infrared signals. When an optical fiber data communication is used, the communication takes place via optical signals that are transmitted on an optical fiber.

In this example the patient monitoring system 1000 may include a display device 1030. The one or more processors 1020 may output, to the display device 1030, the one or more PPG signals, or the blood oxygen saturation signal, or the heart rate signal, or the respiration rate signal and/or the warning signal, respectively.

The display device may be any type of display device, such as a (patient) monitor, a display of a mobile computing device, a virtual! augmented reality display device, and so forth. In general, the output may be provided as a single value, or as a sequence of values over time.

In another example, a patient monitoring system 1000 that includes a nasal interface device 100, 200, 300, 400, 500, 600, and a controller 1010, is provided. The nasal interface device comprises: a clamp 110 for attachment to a nasal alar, or a nasal septum, of a subject; and a measurement unit 120. The measurement unit 120 is mechanically coupled to the clamp such that when the clamp is attached to the nasal alar, or the nasal septum, of the subject, the measurement unit interfaces with the nasal alar, or the nasal septum, respectively for measuring one or more photoplethysmography, PPG, signals for the subject. The controller 1010 comprises one or more processors 1020 configured to:
receive the one or more PPG signals; and extract, from the one or more PPG signals, a heart rate signal and/or a respiration rate signal;
calculate, and output, a warning signal based on the heart rate signal and/or the respiration rate signal.

In this example, by providing the warning signal based on the heart rate signal and/or the respiration rate signal, and which are derived from the PPG signals, the warning signal may be provided in a more straightforward manner.

In another example, the patient monitoring system 1000 includes a fifth measurement unit (not illustrated). In this example, the patient monitoring system 1000 may be provided by any of the patient monitoring systems described above. The fifth measurement unit is configured to measure one or more physiological signals and/or one or more motion signals for the subject. In this example, the one or more processors 1020 are further configured to:
receive the one or more physiological signals and/or the one or more motion signals generated by the fifth measurement unit; and
output the one or more physiological signals and/or the one or more motion signals generated by the fifth measurement unit;
   and/or;
calculate the warning signal based on the one or more physiological signals and/or the one or more motion signals generated by the fifth measurement unit.

The fifth measurement unit may provide physiological signals in a similar manner to the second measurement unit 130 described above. The fifth measurement unit may include one or more motion sensors, such as an accelerometer, or a gyroscope, in order to provide the motion signal(s). The physiological and/or motion signal(s) provided by this example provide further information relating to the health of the subject. By providing the warning signal based on the physiological and/or motion signal(s), a sensitivity, or a specificity of the warning signal may be improved.

In another example, the patient monitoring system 1000 includes at least one wireless communication unit (not illustrated). In this example, the patient monitoring system 1000 may be provided by any of the patient monitoring systems described above. The at least one wireless communication unit is configured to wirelessly transmit to the controller, the signals generated by at least one measurement unit. The wireless transmission of these signal may facilitate a more straightforward interface with the subject.

In another example, a nasal interface device 700, 800 is provided. The nasal interface includes a clamp 110 for attachment to a nasal alar, or a nasal septum, of a subject; and a measurement unit 120. The clamp 110 comprises: a hinge 110a; and two prongs 110b, 110c. The prongs 110b, 110c are mechanically coupled to one another via the hinge 1 10a. The prongs are configured to attach the clamp to the nasal alar, or the nasal septum, of the subject, respectively. The measurement unit 120 is mechanically coupled to one or more of the prongs such that when the prongs are attached to the nasal alar, or the nasal septum, respectively, the measurement unit interfaces with the nasal alar, or the nasal septum, respectively, for measuring one or more photoplethysmography, PPG, signals for the subject. Each prong comprises a curved edge, or a bevelled edge 110b', 110c', for sliding the prong over the nasal alar, or a nasal septum, respectively.

In this example, the curved, or bevelled edges of the prongs of the nasal interface device may facilitate a more straightforward application of the nasal interface device to the nasal alar, or the nasal septum.

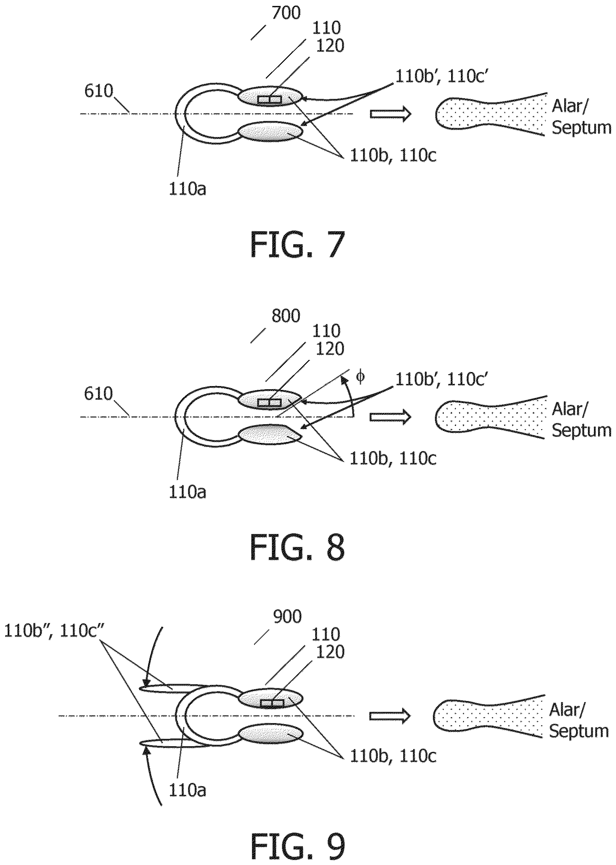

An example of a nasal interface device 700 with prongs that include curved edges is illustrated in Fig. 7. Fig. 7 is a schematic diagram illustrating an example of a nasal interface device 700 with a measurement unit 120 and two prongs 110b, 110c, that include curved edges for sliding over a nasal alar, or a nasal septum, in accordance with some aspects of the present disclosure. The prongs may be formed from, or include, a flexible, or elastic, material such as a polymer, for example. The hinge may provide a restoring force that counteracts a deformation of the hinge from a relaxed state of the hinge. The hinge may be formed from, or include, a flexible, or elastic, material such as a polymer, or a metal, for example. The nasal interface device 700 may be used in combination with any of the patient monitoring systems described above. The measurement unit 120 may be provided in a similar manner to the first measurement unit 120 described above in relation to Fig. 1.

In the example nasal interface 700 illustrated in Fig. 7, the curved edge may have a radius of curvature that facilitates the sliding of the prong over the nasal alar, or a nasal septum. The radius of curvature may be with a range of values in order to facilitate the sliding of the prong over the nasal alar, or a nasal septum.

An example of a nasal interface device 800 with prongs that include bevelled edges is illustrated in Fig. 8. Fig. 8 is a schematic diagram illustrating an example of a nasal interface device 800 with a measurement unit 120 and two prongs 110b, 110c, that include bevelled edges for sliding over a nasal alar, or a nasal septum, in accordance with some aspects of the present disclosure. As in the example illustrated in Fig. 7, the prongs may be formed from, or include, a flexible, or elastic, material such as a polymer, for example. The hinge may provide a restoring force that counteracts a deformation of the hinge from a relaxed state of the hinge. The hinge may be formed from, or include, a flexible, or elastic, material such as a polymer, or a metal, for example. The nasal interface device 800 may be used in combination with any of the patient monitoring systems described above. The measurement unit 120 may be provided in a similar manner to the first measurement unit 120 described above in relation to Fig. 1.

In the example, nasal interface 800 illustrated in Fig. 8, a bevel angle, ϕ, defined between a tangent to a front surface of each prong and a centerline 610 extending between the prongs may be in the range from approximately 10 degrees to approximately 50 degrees. The term approximately is defined herein to represent within ± 10% of these values. The bevel angle ϕ may alternatively be in the range from approximately 20 degrees to approximately 50 degrees, or in the range from approximately 10 degrees to approximately 40 degrees, or in the range from approximately 20 degrees to approximately 40 degrees, or in the range from approximately 25 degrees to approximately 40 degrees, or in the range from approximately 20 degrees to approximately 35 degrees, or in the range from approximately 25 degrees to approximately 35 degrees, or in the range from approximately 20 degrees to approximately 30 degrees, or in the range from approximately 25 degrees to approximately 30 degrees, or in the range from approximately 20 degrees to approximately 25 degrees.

In another example, a nasal interface device 900 is provided. The nasal interface device 900 comprises: a clamp 110 for attachment to a nasal alar, or a nasal septum, of a subject; and a measurement unit 120. The clamp 110 comprises: a hinge 110a; and two prongs 110b, 110c. The prongs are mechanically coupled to one another via the hinge. The prongs are configured to attach the clamp to the nasal alar, or the nasal septum, of the subject, respectively. The measurement unit 120 is mechanically coupled to one or more of the prongs such that when the prongs are attached to the nasal alar, or the nasal septum, respectively, the measurement unit interfaces with the nasal alar, or the nasal septum, respectively, for measuring one or more photoplethysmography, PPG, signals for the subject. Each prong 110b, 110c further comprises a wing 110b", 110c", the wing being mechanically coupled to the corresponding prong, and extending in an opposing direction to the prong. The wings 1 10b", 110c" extend to a position beyond the hinge such that when the prongs are pinched together, the wings pivot about the hinge, opening the hinge for sliding the nasal clamp over the nasal alar, or the nasal septum, respectively.

In this example, the wings of the nasal interface device may facilitate a more straightforward application of the nasal interface device to the nasal alar, or the nasal septum.

An example of the nasal interface device 900 is illustrated in Fig. 9, which is a schematic diagram illustrating an example of a nasal interface device 900 with a measurement unit 120 and wings 110"b, 110"c, for use in sliding over a nasal alar, or a nasal septum, in accordance with some aspects of the present disclosure. The wings 110"b, 110"c, may be formed from, or include, a flexible, or elastic, material such as a polymer, for example.

In another example, a combination of the nasal interface device 700 illustrated in Fig. 7 or Fig. 8, and the nasal interface device 900 illustrated in Fig. 9, is provided. In this example, the nasal interface device 700 illustrated in Fig. 7, or the nasal interface device 800 illustrated in Fig. 8, includes the wings 110"b, 110"c illustrated in Fig. 9.

Various Examples of the present disclosure are listed below.

Example 1. A nasal interface device (100, 200, 300, 400, 500, 600) comprising:
a clamp (110) for attachment to a nasal alar, or a nasal septum, of a subject;
a first measurement unit (120); and
a second measurement unit (130);
wherein the first measurement unit (120) is mechanically coupled to the clamp (110) such that when the clamp is attached to the nasal alar, or the nasal septum, respectively, the first measurement unit (120) interfaces with the nasal alar, or the nasal septum, respectively, for measuring one or more photoplethysmography, PPG, signals for the subject; and
wherein the second measurement unit (130) is configured to measure one or more physiological signals for the subject, the one or more physiological signals being signals other than a PPG signal.
Example 2. The nasal interface device (100) according to Example 1, wherein the second measurement unit is mechanically coupled to the clamp such that when the clamp is attached to the nasal alar, or the nasal septum, respectively, the second measurement unit is positioned in a nasal airflow of the subject for measuring the one or more physiological signals from the nasal airflow of the subject.

Example 3. The nasal interface device (200, 300) according to Example 1, wherein the second measurement unit is mechanically coupled to the clamp such that when the clamp is attached to the nasal alar, or the nasal septum, respectively, the second measurement unit is positioned in an oral airflow of the subject for measuring the one or more physiological signals from the oral airflow of the subject.

Example 4. The nasal interface device (200, 300) according to Example 2 or Example 3, further comprising an arm (140), and wherein the arm comprises a proximal portion and a distal portion;
wherein the proximal portion of the arm is mechanically coupled to the clamp; and
wherein the second measurement unit is arranged on the distal portion of the arm such that when the clamp is attached to the nasal alar, or the nasal septum, respectively, the second measurement unit is positioned in a nasal airflow of the subject, or in an oral airflow of the subject, for measuring the one or more physiological signals from the nasal airflow of the subject, or from the oral airflow of the subject.

Example 5. The nasal interface device (300) according to Example 3, or Example 4 when dependent on Example 3, further comprising a third measurement unit (150);
wherein the third measurement unit is configured to measure one or more physiological signals for the subject, the one or more physiological signals being signals other than a PPG signal; and
wherein the third measurement unit is mechanically coupled to the clamp such that when the clamp is attached to the nasal alar, or the nasal septum, respectively, the third measurement unit is positioned in a nasal airflow of the subject for measuring the one or more physiological signals from the nasal airflow of the subject.

Example 6. The nasal interface device (400, 500) according to Example 1, wherein the first measurement unit comprises one or more electrical conductors (160), or one or more optical waveguides, configured to communicate signals to the first measurement unit (120) for generating the one or more PPG signals, and to communicate the one or more PPG signals generated in response to the signals; and
wherein the nasal interface device further comprises an ear attachment (170) for attachment to an ear of the subject; and
wherein the one or more electrical conductors (160), or the one or more optical waveguides, are mechanically coupled to the ear attachment (170) and the ear attachment is configured to guide the respective one or more electrical conductors, or the one or more optical waveguides, around a portion of the ear of the subject; and
wherein the second measurement unit (130) is mechanically coupled to the ear attachment such that when the ear attachment is attached to the ear of the subject, the second measurement unit interfaces with a region of the ear, or a region of the head of the subject, for measuring the one or more physiological signals for the subject based on a temperature of the region of the ear, or a temperature of the region of the head, respectively.

Example 7. The nasal interface device (400, 500) according to Example 6, wherein the second measurement unit (130) is configured to contact the region of the ear, or wherein the second measurement unit is separated from the region of the ear such that the second measurement unit receives thermal radiation from the region of the ear.

Example 8. The nasal interface device (500) according to Example 7, wherein the second measurement unit (130) comprises an infrared radiation sensor, and wherein the infrared radiation sensor is configured to receive the thermal radiation from the tympanic membrane of the ear of the subject.

Example 9. The nasal interface device (600) according to Example 1, further comprising a cannula (180) for delivering a gas to a nasal cavity of the subject; and
wherein the first measurement unit (120) comprises one or more electrical conductors (160), or one or more optical waveguides, configured to communicate signals to the first measurement unit for generating the one or more PPG signals, and to communicate the one or more PPG signals generated in response to the signals; and
wherein the cannula (180) is mechanically coupled to the clamp (110), and wherein the cannula is configured to support the one or more electrical conductors (160), or one or more optical waveguides along a path of the one or more electrical conductors, or the one or more optical waveguides, respectively; and
wherein the second measurement unit (130) is mechanically coupled to the clamp, or to the cannula, such that when the clamp is attached to the nasal alar, or the nasal septum, respectively, the second measurement unit is positioned in a nasal airflow of the subject for measuring the one or more physiological signals from the nasal airflow of the subject.

Example 10. The nasal interface device (100, 200, 300, 400, 500) according to any one of Examples 1-8, further comprising a cannula (180) for delivering a gas to a nasal cavity of the subject;
wherein the cannula is mechanically coupled to the clamp (110) such that when the clamp is attached to the nasal alar, or the nasal septum, respectively, a gas delivery port of the cannula is positioned in a nasal airflow of the subject for delivering the gas to the nasal cavity of the subject.

Example 11. The nasal interface device (100, 200, 300, 400, 500, 600) according to any previous Example, wherein the physiological signals include one or more of: a temperature signal representing a temperature of the subject, a temperature signal representing a temperature of exhaled breath of the subject, an air flow signal representing a respiration rate of the subject, and a carbon dioxide signal representing a carbon dioxide content of exhaled breath of the subject.

Example 12. The nasal interface device (100, 200, 300, 400, 500, 600) according to any previous Example, wherein the measurement unit (120), or the first measurement unit (120), comprises at least one optical source and at least one optical detector; and wherein the at least one optical source and at least one optical detector are configured to measure one or more of the following optical parameters for a portion of the nasal alar, or a portion of the nasal septum, respectively: an optical transmission, an optical reflectance, an optical absorption, and an optical scattering.

Example 13. The nasal interface device according to any previous Example, wherein the second measurement unit (130) or the third measurement (150) comprises at least one source and/or at least one detector.

Example 14. The nasal interface device (100, 200, 300, 400, 500, 600) according to any previous Example, further comprising a fourth measurement unit;
wherein the fourth measurement unit is mechanically coupled to the clamp, or to the arm, or to the ear attachment, or to the cannula; and
wherein the fourth measurement unit is configured to measure a temperature of an environment of the subject.

Example 15. A patient monitoring system (1000) comprising:
the nasal interface device (100, 200, 300, 400, 500, 600) according to any one of Examples 1 - 14; and
a controller (1010);
wherein the controller comprises one or more processors (1020) configured to:
   receive the one or more PPG signals and/or the one or more physiological signals; and
   output the one or more PPG signals;
      and/or;
   calculate, and output, a blood oxygen saturation signal, or a heart rate signal, or a respiration rate signal, based on the one or more PPG signals and/or based on the one or more physiological signals;
      and/or
   calculate, and output, a warning signal based on the one or more PPG signals and/or based on the one or more physiological signals.

Example 16. The patient monitoring system (1000) according to Example 15, further comprising a display device (1030); and
wherein the one or more processors (1020) are further configured to output, to the display device (1030), the one or more PPG signals, or the blood oxygen saturation signal, or the heart rate signal, or the respiration rate signal and/or the warning signal, respectively.

Example 17. A patient monitoring system (1000) comprising:
a nasal interface device (100, 200, 300, 400, 500, 600); and
a controller (1010);
wherein the nasal interface device comprises:
   a clamp (110) for attachment to a nasal alar, or a nasal septum, of a subject; and
   a measurement unit (120); and
   wherein the measurement unit (120) is mechanically coupled to the clamp such that when the clamp is attached to the nasal alar, or the nasal septum, of the subject, the measurement unit interfaces with the nasal alar, or the nasal septum, respectively for measuring one or more photoplethysmography, PPG, signals for the subject; and
wherein the controller (1010) comprises one or more processors (1020) configured to:
   receive the one or more PPG signals; and
   extract, from the one or more PPG signals, a heart rate signal and/or a respiration rate signal;
   calculate, and output, a warning signal based on the heart rate signal and/or the respiration rate signal.

Example 18. The patient monitoring system (1000) according to any one of Examples 15 - 17, further comprising a fifth measurement unit; wherein the fifth measurement unit is configured to measure one or more physiological signals and/or one or more motion signals for the subject; and wherein the one or more processors (1020) are further configured to:
receive the one or more physiological signals and/or the one or more motion signals generated by the fifth measurement unit; and
output the one or more physiological signals and/or the one or more motion signals generated by the fifth measurement unit;
   and/or;
calculate the warning signal based on the one or more physiological signals and/or the one or more motion signals generated by the fifth measurement unit.

Example 19. The patient monitoring system (1000) according to any one of Examples 15 - 18, further comprising at least one wireless communication unit;
wherein the at least one wireless communication unit is configured to wirelessly transmit to the controller, the signals generated by at least one measurement unit.

Example 20. A nasal interface device (700, 800) comprising:
a clamp (110) for attachment to a nasal alar, or a nasal septum, of a subject; and
a measurement unit (120);
wherein the clamp (110) comprises:
   a hinge (110a); and
   two prongs (110b, 110c);
wherein the prongs (1 10b, 1 10c) are mechanically coupled to one another via the hinge (110a);
wherein the prongs are configured to attach the clamp to the nasal alar, or the nasal septum, of the subject, respectively; and
wherein the measurement unit (120) is mechanically coupled to one or more of the prongs such that when the prongs are attached to the nasal alar, or the nasal septum, respectively, the measurement unit interfaces with the nasal alar, or the nasal septum, respectively, for measuring one or more photoplethysmography, PPG, signals for the subject; and
wherein each prong comprises a curved edge, or a bevelled edge (110b', 110c'), for sliding the prong over the nasal alar, or a nasal septum, respectively.

Example 21. The nasal interface device (800) according to Example 20, wherein a bevel angle (ϕ) defined between a tangent to a front surface of each prong and a centerline (610) extending between the prongs is in the range from approximately 10 degrees to approximately 50 degrees.

Example 22. A nasal interface device (900) comprising:
a clamp (110) for attachment to a nasal alar, or a nasal septum, of a subject; and
a measurement unit (120);
wherein the clamp (110) comprises:
   a hinge (110a); and
   two prongs (110b, 110c);
wherein the prongs are mechanically coupled to one another via the hinge;
wherein the prongs are configured to attach the clamp to the nasal alar, or the nasal septum, of the subject, respectively; and
wherein the measurement unit (120) is mechanically coupled to one or more of the prongs such that when the prongs are attached to the nasal alar, or the nasal septum, respectively, the measurement unit interfaces with the nasal alar, or the nasal septum, respectively, for measuring one or more photoplethysmography, PPG, signals for the subject; and
wherein each prong (110b, 110c) further comprises a wing (110b", 110c"), the wing being mechanically coupled to the corresponding prong, and extending in an opposing direction to the prong; and
wherein the wings (110b", 110c") extend to a position beyond the hinge such that when the prongs are pinched together, the wings pivot about the hinge, opening the hinge for sliding the nasal clamp over the nasal alar, or the nasal septum, respectively.

The above examples are to be understood as illustrative of the present disclosure, and not restrictive. Further examples are also contemplated. For instance, features described in relation to one example of a nasal interface device may be implemented in another example of a nasal interface device, in a corresponding manner. Similarly, features described in relation to an example of a nasal interface device may be included in examples of patient monitoring systems. It is to be understood that a feature described in relation to any one example may be used alone, or in combination with other described features, and may be used in combination with one or more features of another of the examples, or a combination of other examples. Furthermore, equivalents and modifications not described above may also be employed without departing from the scope of the invention, which is defined in the accompanying claims. In the claims, the word "comprising" does not exclude other elements or operations, and the indefinite article "a" or "an" does not exclude a plurality. The mere fact that certain features are recited in mutually different dependent claims does not indicate that a combination of these features cannot be used to advantage. Any reference signs in the claims should not be construed as limiting their scope.

## Claims

1. A nasal interface device (100, 200, 300, 400, 500, 600) comprising:
a clamp (110) for attachment to a nasal alar, or a nasal septum, of a subject;
a first measurement unit (120); and
a second measurement unit (130);
wherein the first measurement unit (120) is mechanically coupled to the clamp (110) such that when the clamp is attached to the nasal alar, or the nasal septum, respectively, the first measurement unit (120) interfaces with the nasal alar, or the nasal septum, respectively, for measuring one or more photoplethysmography, PPG, signals for the subject; and
wherein the second measurement unit (130) is configured to measure one or more physiological signals for the subject, the one or more physiological signals being signals other than a PPG signal.

2. The nasal interface device (100) according to claim 1, wherein the second measurement unit is mechanically coupled to the clamp such that when the clamp is attached to the nasal alar, or the nasal septum, respectively, the second measurement unit is positioned in a nasal airflow of the subject for measuring the one or more physiological signals from the nasal airflow of the subject.

3. The nasal interface device (200, 300) according to claim 1, wherein the second measurement unit is mechanically coupled to the clamp such that when the clamp is attached to the nasal alar, or the nasal septum, respectively, the second measurement unit is positioned in an oral airflow of the subject for measuring the one or more physiological signals from the oral airflow of the subject.

4. The nasal interface device (200, 300) according to claim 2 or claim 3, further comprising an arm (140), and wherein the arm comprises a proximal portion and a distal portion;
wherein the proximal portion of the arm is mechanically coupled to the clamp; and
wherein the second measurement unit is arranged on the distal portion of the arm such that when the clamp is attached to the nasal alar, or the nasal septum, respectively, the second measurement unit is positioned in a nasal airflow of the subject, or in an oral airflow of the subject, for measuring the one or more physiological signals from the nasal airflow of the subject, or from the oral airflow of the subject.

5. The nasal interface device (300) according to claim 3, or claim 4 when dependent on claim 3, further comprising a third measurement unit (150);
wherein the third measurement unit is configured to measure one or more physiological signals for the subject, the one or more physiological signals being signals other than a PPG signal; and
wherein the third measurement unit is mechanically coupled to the clamp such that when the clamp is attached to the nasal alar, or the nasal septum, respectively, the third measurement unit is positioned in a nasal airflow of the subject for measuring the one or more physiological signals from the nasal airflow of the subject.

6. The nasal interface device (400, 500) according to claim 1, wherein the first measurement unit comprises one or more electrical conductors (160), or one or more optical waveguides, configured to communicate signals to the first measurement unit (120) for generating the one or more PPG signals, and to communicate the one or more PPG signals generated in response to the signals; and
wherein the nasal interface device further comprises an ear attachment (170) for attachment to an ear of the subject; and
wherein the one or more electrical conductors (160), or the one or more optical waveguides, are mechanically coupled to the ear attachment (170) and the ear attachment is configured to guide the respective one or more electrical conductors, or the one or more optical waveguides, around a portion of the ear of the subject; and
wherein the second measurement unit (130) is mechanically coupled to the ear attachment such that when the ear attachment is attached to the ear of the subject, the second measurement unit interfaces with a region of the ear, or a region of the head of the subject, for measuring the one or more physiological signals for the subject based on a temperature of the region of the ear, or a temperature of the region of the head, respectively.

7. The nasal interface device (400, 500) according to claim 6, wherein the second measurement unit (130) is configured to contact the region of the ear, or wherein the second measurement unit is separated from the region of the ear such that the second measurement unit receives thermal radiation from the region of the ear.

8. The nasal interface device (500) according to claim 7, wherein the second measurement unit (130) comprises an infrared radiation sensor, and wherein the infrared radiation sensor is configured to receive the thermal radiation from the tympanic membrane of the ear of the subject.

9. The nasal interface device (100, 200, 300, 400, 500, 600) according to any previous claim, wherein the physiological signals include one or more of: a temperature signal representing a temperature of the subject, a temperature signal representing a temperature of exhaled breath of the subject, an air flow signal representing a respiration rate of the subject, and a carbon dioxide signal representing a carbon dioxide content of exhaled breath of the subject.

10. The nasal interface device (100, 200, 300, 400, 500, 600) according to any previous claim, wherein the measurement unit (120), or the first measurement unit (120), comprises at least one optical source and at least one optical detector; and wherein the at least one optical source and at least one optical detector are configured to measure one or more of the following optical parameters for a portion of the nasal alar, or a portion of the nasal septum, respectively: an optical transmission, an optical reflectance, an optical absorption, and an optical scattering.

11. The nasal interface device according to any previous claim, wherein the second measurement unit (130) or the third measurement (150) comprises at least one source and/or at least one detector.

12. The nasal interface device (100, 200, 300, 400, 500, 600) according to any previous claim, further comprising a fourth measurement unit;
wherein the fourth measurement unit is mechanically coupled to the clamp, or to the arm, or to the ear attachment, or to the cannula; and
wherein the fourth measurement unit is configured to measure a temperature of an environment of the subject.

13. A patient monitoring system (1000) comprising:
the nasal interface device (100, 200, 300, 400, 500, 600) according to any one of claims 1 - 12; and
a controller (1010);
wherein the controller comprises one or more processors (1020) configured to:
receive the one or more PPG signals and/or the one or more physiological signals; and
output the one or more PPG signals;
and/or;
calculate, and output, a blood oxygen saturation signal, or a heart rate signal, or a respiration rate signal, based on the one or more PPG signals and/or based on the one or more physiological signals;
and/or
calculate, and output, a warning signal based on the one or more PPG signals and/or based on the one or more physiological signals.

14. The patient monitoring system (1000) according to claim 13, further comprising a display device (1030); and
wherein the one or more processors (1020) are further configured to output, to the display device (1030), the one or more PPG signals, or the blood oxygen saturation signal, or the heart rate signal, or the respiration rate signal and/or the warning signal, respectively.

15. The patient monitoring system (1000) according to any one of claims 13 - 14, further comprising a fifth measurement unit; wherein the fifth measurement unit is configured to measure one or more physiological signals and/or one or more motion signals for the subject; and wherein the one or more processors (1020) are further configured to:
receive the one or more physiological signals and/or the one or more motion signals generated by the fifth measurement unit; and
output the one or more physiological signals and/or the one or more motion signals generated by the fifth measurement unit;
and/or;
calculate the warning signal based on the one or more physiological signals and/or the one or more motion signals generated by the fifth measurement unit.
